# EUROPÄISCHE PATENTSCHRIFT

(11) **EP 1 915 185 B1**
(45) Veröffentlichungstag und Bekanntmachung des Hinweises auf die Patenterteilung: **30.03.2011**
(21) Anmeldenummer: 06776748.3
(22) Anmeldetag: 10.08.2006
(51) Int. Cl.: A61M 5/50, A61M 5/315

(54) **SPRITZE**
SYRINGE
SERINGUE

(30) Priorität: 11.08.2005 DE 102005037962
(43) Veröffentlichungstag der Anmeldung: 30.04.2008
(73) Patentinhaber: Arzneimittel GmbH Apotheker Vetter & Co. Ravensburg, 88212 Ravensburg (DE)
(72) Erfinder: BÖBST, Benjamin, 88441 Mittelbiberach (DE)
(74) Vertreter: Gleiss, Alf-Olav
(86) Internationale Anmeldenummer: PCT/EP2006/007927
(87) Internationale Veröffentlichungsnummer: WO 2007/017281

(56) Entgegenhaltungen:
- WO-A-94/13339
- WO-A-96/39214
- US-A1- 2004 122 361

## Beschreibung

Die Erfindung betrifft eine Spritze mit einem mit einem Medium befüllten Spritzenzylinder und mit einem im Spritzenzylinder beweglich angeordneten Verschlussstopfen.

Spritzen der hier angesprochenen Art sind bekannt. Es ist bekannt, dass bei einem Druckabfall, wie er beispielsweise beim Transport von Spritzen in Flugzeugen vorkommt, der Verschlussstopfen in befüllten Spritzen wandert, selbst wenn in dem im Spritzenzylinder vorhandenen Medium nur eine kleine Luftblase vorhanden ist. Dies führt dazu, dass die Sterilität der im Spritzenzylinder vorhandenen Produkte beeinträchtigt werden kann.

Eine aus dem Stand der Technik bekannte Spritze ist im Dokument WO96/39214 offenbart. Der Oberbegriff des Anspruchs 1 ist auf diesem Dokument basiert.

Aufgabe der Erfindung ist es daher, eine Spritze zu schaffen, die diesen Nachteil nicht aufweist, also die Sterilität der im Spritzenzylinder vorhandenen Produkte gewährleistet.

Zur Lösung dieser Aufgabe wird eine Spritze vorgeschlagen, die die in Anspruch 1 genannten Merkmale umfasst. Die Spritze weist einen mit einem Medium befüllten Spritzenzylinder auf, außerdem einen innerhalb des Spritzenzylinders beweglichen Verschlussstopfen, der dazu dient, das Medium auf bekannte Weise bei Bedarf aus dem Spritzenzylinder auszutragen. Die Spritze zeichnet sich durch eine Sicherung aus, die ein Zurückgleiten des Verschlussstopfens im Spritzenzylinder verhindert. Es ist also auch bei einem Druckabfall nicht möglich, dass sich der Verschlussstopfen bewegt und dabei Flächen der Innenseite des Spritzenzylinders überstreicht, die möglicherweise kontaminiert sind. Aufgrund dieser Sicherung ist es nicht mehr möglich, dass das in den Spritzenzylinder eingebrachte und von dem Verschlussstopfen abgeschlossene Medium verunreinigt wird. Bevorzugt wird ein Ausführungsbeispiel der Erfindung, das sich dadurch auszeichnet, dass die Sicherung ein Sperrelement umfasst, das an einer mit dem Verschlussstopfen zusammenwirkenden Kolbenstange angreift. Damit ist eine relativ einfache Sicherung realisierbar, weil die Kolbenstange leichter zugänglich ist als der Verschlussstopfen selbst.

Bevorzugt wird weiterhin ein Ausführungsbeispiel der Erfindung, das sich dadurch auszeichnet, dass das Sperrelement verkippbar ist. Es kann damit einerseits eine Freigabeposition einnehmen, wenn der Verschlussstopfen durch die Kolbenstange in den Spritzenzylinder hineinverlagert wird. Andererseits kann das Sperrelement aus der Freigabeposition in eine Verriegelungsposition verkippen, wenn der Verschlussstopfen und damit die Kolbenstange eine entgegengesetzte Bewegung ausführen wollen. Dabei ist vorgesehen, dass das Sperrelement mit der Mittelachse der Kolbenstange einen Winkel < 90° einschließt, also gegenüber der Mittelachse der Spritze schräg gestellt ist. Bei einer Änderung dieses Winkels bewegt sich das Sperrelement aus einer Freigabeposition in eine Verriegelungsposition und umgekehrt. Die Spritze ist sehr einfach aufgebaut und damit störungsunanfällig.

Besonders bevorzugt wird ein Ausführungsbeispiel der Spritze, das sich dadurch auszeichnet, dass das Sperrelement mit einer Vorspannkraft beaufschlagbar ist, die es in die Verriegelungsposition drängt. Damit wird eine praktisch spielfreie Sicherung des Verschlussstopfens gegen eine ungewollte Bewegung sichergestellt.

Besonders bevorzugt wird ein weiteres Ausführungsbeispiel der Spritze, das sich dadurch auszeichnet, dass das Sperrelement als Ring ausgebildet ist. Es kann damit sichergestellt werden, dass das Sperrelement die Kolbenstange an zwei Seiten so erfasst, dass ein Zurückgleiten des Verschlussstopfens sicher vermieden wird. Besonders bevorzugt wird ein Ausführungsbeispiel der Spritze, das sich dadurch auszeichnet, dass die Sicherung ein Entsperrelement umfasst, mit dessen Hilfe das Sperrelement in die Freigabeposition verlagerbar und/oder in dieser gehalten werden kann. Trotz der ein ungewolltes Zurückgleiten des Verschlussstopfen verhindernden Sicherung ist es also sehr wohl möglich, den Verschlussstopfen bei Bedarf gezielt zu verlagern, insbesondere zurückzuziehen.

Weitere Ausgestaltungen ergeben sich aus den Unteransprüchen.

Die Erfindung wird im Folgenden anhand der Zeichnung näher erläutert. Es zeigen:
Figur 1 eine Prinzipskizze einer Spritze in perspektivischer Darstellung,
Figur 2 ein erstes Ausführungsbeispiel einer Sicherung einer Spritze gemäß Figur 1 und
Figur 3 ein zweites Ausführungsbeispiel einer Sicherung.

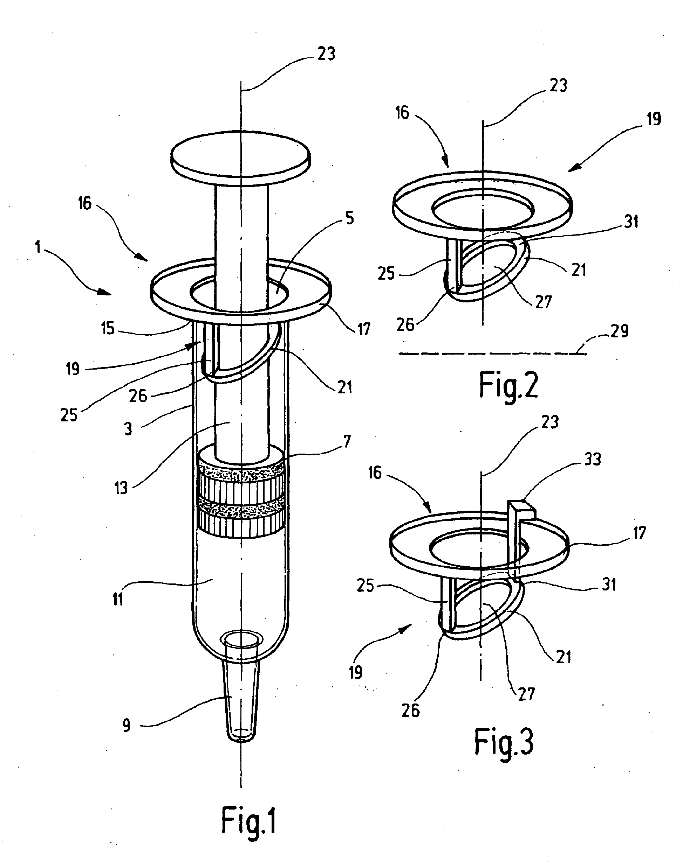

Die in Figur 1 dargestellte Spritze 1 weist einen Spritzenzylinder 3 auf, in dessen Innenraum 5 ein Verschlussstopfen 7 beweglich verlagerbar eingesetzt ist. An seinem einen, in Figur 1 unteren, Ende ist der Spritzenzylinder 3 mit einem Nadelansatz 9 versehen. Dieser kann bei Bedarf mit einer geeigneten Kappe verschlossen werden, wenn in den Spritzenzylinder 3 ein Medium eingebracht ist. Unterhalb des Verschlussstopfens 7 kann damit ein Bereich 11 zur Aufnahme eines Mediums gebildet werden.

An dem Verschlussstopfen 7 greift eine Kolbenstange 13 an, die oben aus dem dem Nadelansatz 9 gegenüberliegenden offenen Ende des Spritzenzylinders 1 hinausragt. Der Bereich 11, der der Aufnahme eines Mediums dient, ist gegenüber dem offenen Ende 15 durch den Verschlussstopfen 7 abgeschlossen, so dass das Medium bei verschlossenem Nadelansatz 9 gegen Umwelteinflüsse, insbesondere gegen Kontamination gesichert ist.

Am oberen Ende 15 des Spritzenzylinders 3 ist ein Verschlusselement 16 vorgesehen, das am Spritzenzylinder 3 angesetzt und vorzugsweise fest angebracht ist. Das Verschlusselement 16 ist vorzugsweise als so genannter Backstop ausgebildet und verhindert seinerseits ein Herausgleiten des Verschlussstopfens 7 aus dem Spritzenzylinder 3. Bei dem hier dargestellten Ausführungsbeispiel ist das Verschlusselement 16 zur Verbesserung der Handhabbarkeit der Spritze als Fingerauflage 17 ausgebildet.

Die Spritze 1 umfasst weiterhin eine Sicherung 19, die dazu dient, ein ungewolltes Zurückgleiten des Verschlussstopfens 7 im Inneren des Spritzenzylinders 3 zu verhindern. Sie bewirkt also, dass der Verschlussstopfen 7 ungewollt in Richtung zum offenen Ende 15 der Spritze 1 verlagert werden kann.

Bei dem hier dargestellten Ausführungsbeispiel wirkt die Sicherung 19 mit der Kolbenstange 13 zusammen. Sie weist ein Sperrelement 21 auf, das gegenüber der Mittelachse 23 der Spritze 1, die auch die Mittelachse der Kolbenstange 13 bildet, geneigt ist und an der Kolbenstange 13 angreift. Die Sicherung 19 weist außerdem eine Halterung 25 für das Sperrelement 21 auf. Aus Figur 1 ist ersichtlich, dass das Sperrelement 21 an einem Anlenkpunkt 26 mit der Halterung 25 verbunden ist, und dass dieser tiefer liegt, also näher an dem Verschlussstopfen 7, als das gegenüberliegende Ende des Sperrelements 21. Die Halterung 25 hält das Sperrelement 21 in der hier dargestellten, gegenüber der Mittelachse 23 geneigten Stellung.

Aus Figur 1 ist ersichtlich, dass die Halterung 25 das Sperrelement 21 ausschließlich am Anlenkpunkt 26 hält, sodass diese frei um den Anlenkpunkt 26 schwenkbar ist.

In Figur 1 ist die Halterung 25 als parallel zur Mittelachse 23 der Spritze 1 verlaufender Steg dargestellt, er erstreckt sich damit geradlinig in Richtung des Verlagerungsweges sowohl des Verschlussstopfens 7 als auch der Kolbenstange 13. Die Nachgiebigkeit der Halterung 25 in Richtung der Mittelachse 23 ist also relativ gering. Es ist aber auch denkbar, die Halterung 25 nachgiebig auszubilden, beispielsweise einen in Wellenlinien verlaufenden Steg einzusetzen. Dieser kann bei von unten, also aus der Richtung des Nadelansatzes 9, wirkenden Kräften etwas nachgeben, sodass der Anlenkpunkt 26 nicht überlastet wird und das Sperrelement 21 möglicherweise von der Halterung 25 abreißt.

Die Halterung ihrerseits kann Teil der Fingerauflage 17 oder an dieser befestigt sein. Sie ist vorzugsweise so ausgebildet, dass sie das Sperrelement 21 in die hier dargestellte geneigte Stellung drängt, also mit einer Vorspannkraft beaufschlagt.

Die Vorspannkraft kann auf einfache Weise dadurch realisiert werden, dass das Sperrelement 21 in einer vorgegebenen Position an der Halterung 25 befestigt wird, sodass die Halterung 25 das Sperrelement 21 in eine bestimmte Position drängt. Beispielsweise ist es möglich, die Halterung 25 und das Sperrelement 21 aus einem Stück herzustellen, insbesondere im Kunst-Spritzgussverfahren, um eine definierte Position des Sperrelements 21 gegenüber der Halterung 25 vorzugeben.

Diese Art der Realisierung einer Vorspannkraft ist besonders preiswert. Insbesondere bedarf es nicht irgendwelcher Federelemente, die das Sperrelement 21 in eine vorgegebene Position drängen. Dies führt, wie gesagt, dazu, dass sich ein besonders einfacher und kostengünstiger Aufbau der Sicherung 19 ergibt.

Die hier erwähnte Vorspannkraft führt dazu, dass das Sperrelement 21 der Sicherung 19 selbsttätig eine Verriegelungsposition einnimmt, die ein Zurückgleiten der Kolbenstange 13 verhindert, unabhängig davon, in welcher Position sich die Kolbenstange innerhalb des Spritzenzylinders 3 befindet. Damit wird der Verschlussstopfen 7 durch das Sperrelement 21 ebenfalls in jeder Position, die er innerhalb des Spritzenzylinders 3 annimmt, an einem Zurückgleiten gehindert. Besonders vorteilhaft ist es, dass, wie oben erläutert, dieses Zurückgleiten selbsttätig verhindert wird, ohne dass es irgendwelcher zusätzlicher Federelemente oder dergleichen bedürfte.

Grundsätzlich ist es auch möglich, die Halterung 25, die hier einen parallel zur Mittelachse 23 verlaufenden Steg bildet, an der Innenwand des Spritzenzylinders 3 zu befestigen, beispielsweise anzukleben oder dergleichen. Es hat sich aber als besonders günstig erwiesen, das Verschlusselement 16, hier die Fingerauflage 17, mit der Sicherung 19 zu kombinieren, vorzugsweise aus einem Stück herzustellen.

Figur 2 zeigt das erste Ausführungsbeispiel der Sicherung 19 separat, also ohne den Spritzenzylinder 3, die Kolbenstange 13 und den Verschlussstopfen 7. Gleiche Teile sind mit gleichen Bezugsziffern versehen, so dass insofern auf die Beschreibung zu Figur 1 verwiesen wird.

Hier wird deutlich, dass das Sperrelement 21 der Sicherung als geschlossener Ring ausgebildet ist, der an der Halterung 25 ansetzt und gegenüber der Mittelachse 23 geneigt angeordnet ist. Das Sperrelement 21 umschließt einen Innenraum 27, hier also eine Kreisfläche, die geneigt zur Mittelachse 23 angeordnet ist.

Wird das Sperrelement 21 nach unten gedrängt, also um den Anlenkpunkt 26 an der Halterung 25 im Uhrzeigersinn nach unten verschwenkt, so vergrößert sich die Projektion des Innenraums 27 auf eine gedachte Ebene 29, auf der die Mittelachse 23 senkrecht steht. Durch diesen aufgrund der Schwenkbewegung vergrößerten Innenraum 27 kann die Kolbenstange 13 nach unten verlagert werden, also in Figur 1 in Richtung auf den Nadelansatz 9. Die Schwenkbewegung des Sperrelements 21 nach unten in eine Freigabeposition erfolgt hier durch die Reibungskräfte zwischen dem Sperrelement 21 und der dieses durchdringenden Kolbenstange 13. Es zeigt sich hier, dass das Sperrelement 21 selbsttätig bei einer Verlagerung der Kolbenstange 13 in Richtung auf den Nadelansatz 19 in seien Freigabeposition verschwenkt wird. Es bedarf also keiner zusätzlicher Maßnahmen oder Betätigungselemente, das Sperrelement 21 in diese Freigabeposition zu verlagern.

Wird die Kolbenstange 13 in entgegengesetzter Richtung nach oben verlagert, schwenkt das Sperrelement 21 aufgrund von Reibungskräften zwischen dem Sperrelement 21 und der Kolbenstange 13 und insbesondere durch die Vorspannkraft der Halterung 25 um den Anlenkpunkt 26 zur Halterung 25 gegen den Uhrzeigersinn nach oben. Dadurch wird die Projektion des Innenraums 27 auf die Ebene 29 kleiner, das Sperrelement 21 verhakt sich also mit der Kolbenstange 13, so dass eine Aufwärtsbewegung nach oben, also vom Nadelansatz 9 weg, verhindert wird. Wird also das Sperrelement 21 um den Anlenkpunkt 26 an der Halterung 25 gegen den Uhrzeigersinn nach oben verschwenkt, gelangt das Sperrelement 21 in seine Verriegelungsposition.

Aus Figur 2 ist ersichtlich, dass das Sperrelement 21 mit seinem dem Anlenkpunkt 26 an der Halterung 25 abgewandten Ende 31 an der Außenfläche der Kolbenstange 13 eingreift, wenn es in seine Verriegelungsposition nach oben geschwenkt ist. In der Verriegelungsposition kann sich das Sperrelement 21 auch im Bereich des Anlenkpunkts 26 an der Kolbenstange 13 anlegen und verhaken. Die Halterung 25 ist hier so ausgebildet, dass das Sperrelement 21 durch Vorspannkräfte in die Verriegelungsposition gedrängt wird.

Es wird deutlich, dass das Sperrelement 21 nicht, wie hier dargestellt, als geschlossener Ring ausgebildet zu sein braucht. Es reicht, wenn das Sperrelement 21 ausgehend vom Anlenkpunkt 26 an der Halterung 25 schräg nach oben ragt und auf der dem Anlenkpunkt 26 gegenüberliegenden Seite der Außenseite der Kolbenstange 13 an dieser angreift, sobald das Sperrelement 21 in seine Verriegelungsposition gegen den Uhrzeigersinn verschwenkt und/oder von der Vorspannkraft gedrängt wird. Es könnte also an Stelle eines geschlossenen Rings mit kreisförmiger oder elliptischer Kontur auch ein C-förmiges Sperrelement 21 realisiert werden, oder aber ein Sperrelement, das drei im Wesentlichen U-förmige Schenkel aufweist, von denen zwei im Wesentlichen parallel zu einer Basis verlaufen. Einer der parallelen Schenkel ist am Anlenkpunkt 26 der Halterung 25 angesetzt, der andere greift an der dem Anlenkpunkt 26 gegenüberliegenden Seite an der Außenfläche der Kolbenstange 13 an, um in der Verriegelungsposition des Sperrelements 21 eine Verlagerung der Kolbenstange nach oben zu verhindern.

Die Erläuterungen zu dem Sperrelement 21 zeigen, dass vorzugsweise die Außenkontur der Kolbenstange 13 und die Innenkontur des Sperrelements 21 aufeinander abgestimmt werden, um optimale Haltekräfte zu erreichen, wenn das Sperrelement 21 in seine Verriegelungsposition verschwenkt wird. Die Kolbenstange 13 kann aus mehreren Einzelstegen zusammengesetzt sein. Bekannt ist es beispielsweise, vier senkrecht aufeinander stehende Stege zur Realisierung des Grundkörpers der Kolbenstange 13 vorzusehen. Es können aber auch mehr als vier derartige Stege eingesetzt werden. Außerdem kann die Kolbenstange 13 aus einem Voll- oder Hohlkörper hergestellt werden, der eine kreisförmige oder ovale Kontur zeigt. Aus den Erläuterungen wird Folgendes klar: Das Sperrelement 21 wird von der Halterung 26 so gehalten, dass es vorzugsweise in seine Verriegelungsposition gedrängt wird. In dieser kann die Kolbenstange 13 nicht ungewollt nach oben, also in Figur 1 vom Nadelansatz 9 weg, verlagert werden. Wird an der Kolbenstange 13 gezogen, so verschwenkt dadurch das Sperrelement 21 um den Anlenkpunkt 26 weiter nach oben, was den Innenraum 27 verkleinert und zu einer erhöhten Haltekraft an der Kolbenstange 13 führt. Bei einer Bewegung der Kolbenstange nach unten wird das Sperrelement 21 um den Anlenkpunkt 26 im Uhrzeigersinn verschwenkt, weil die Außenseite der Kolbenstange 13 an der Innenfläche des Sperrelements 21 Reibungskräfte aufbaut. Durch diese Schwenkbewegung vergrößert sich die Projektion des Innenraums 27 auf die Ebene 29, also der Freiraum innerhalb des Sperrelements 21; dieses wird also bei einer Bewegung der Kolbenstange 13 nach unten in seine Freigabestellung verschwenkt.

Figur 3 zeigt ein abgewandeltes Ausführungsbeispiel der Sicherung 19. Gleiche Teile sind mit gleichen Bezugsziffern versehen, so dass auf die Beschreibung zu den Figuren 1 und 2 verwiesen wird.

Der einzige Unterschied der Sicherung 19 gemäß Figur 3 gegenüber der Sicherung 19 gemäß Figur 2 besteht darin, dass an dem Sperrelement 21 ein Entsperrelement 33 vorgesehen ist. Wird von oben auf dieses eine Kraft ausgeübt, so wird das Sperrelement 21 um den Anlenkpunkt 26 nach unten, also im Uhrzeigersinn, verschwenkt und in seine Freigabeposition verlagert. Ist die auf das Entsperrelement 33 ausgeübte Kraft größer als die Vorspannkraft und die Reibungskräfte zwischen der Außenseite der Kolbenstange 13 und der Innenseite des Sperrelements 21, so wird das Sperrelement 21 in der Freigabeposition gehalten. Damit kann die Kolbenstange 13 bei Bedarf auch nach oben verlagert werden, also in Richtung auf das offene Ende 15 des Spritzenzylinders 13, auch wenn die Sicherung 19 in diesen eingesetzt ist.

Das Entsperrelement 33 erlaubt es also, die selbsttätige Wirkung der Sicherung 19, die ein ungewolltes nach oben Verschieben der Kolbenstange 13 und damit des Verschlussstopfens 7 verhindert, aufzuheben und den Verschlussstopfen 7 mit der Kolbenstange 13 nach oben zu ziehen. Dadurch, dass das Verschlusselement 16 als Backstop ausgelegt ist, kann auch bei Entriegelung der Sicherung 19 der Verschlussstopfen 7 nicht ganz aus dem Spritzenzylinder 3 herausgezogen werden.

Vorzugsweise greift das Entsperrelement 33 am freien Ende 31 des Sperrelements 21 an, also auf der dem Anlenkpunkt 26 gegenüberliegenden Seite. Dadurch werden die zur Entriegelung des Sperrelements 21 erforderlichen Kräfte auf ein Minimum reduziert.

Bei dem in Figur 3 dargestellten Ausführungsbeispiel ragt das Entsperrelement 33 über die Fingerauflage 17 nach oben hinaus. Es ist also für einen Benutzer der Spritze 1 frei zugänglich. Denkbar ist es auch, das dem Sperrelement 21 abgewandte freie Ende des Entsperrelements 33 noch in den Innenraum 5 des Spritzenzylinders 3 zu verlegen, so dass eine Entsperrung der Sicherung 19 nur mittels eines Werkzeugs möglich ist. Dies erhöht die Sicherheit der hier beschriebenen Sicherung 19.

Die Sicherung 19 ist vorzugsweise aus Kunststoff hergestellt und kann insbesondere im Spritzgussverfahren hergestellt werden. Dies senkt die Kosten für die Herstellung der Sicherung 19 wesentlich. Besonders günstig ist es, wenn die Fingerauflage 17 und die Sicherung 19 beide aus dem gleichen Material hergestellt und einstückig realisiert sind.

Grundsätzlich ist es aber sehr wohl möglich, das Sperrelement 21 aus Metall herzustellen oder aus einem Verbundmaterial, um einen besonders guten Eingriff auf der Außenseite der Kolbenstange 13 zu realisieren. Damit wird eine besonders hohe Sicherheit gegen eine ungewollte Verlagerung des Verschlussstopfens 7 und der Kolbenstange 13 gewährleistet.

Um den Eingriff des Sperrelements 21 auf der Außenseite der Kolbestange 13 zu verbessern, ist es möglich, die Außenfläche der Kolbenstange 13 mit einer gewissen Rauhigkeit auszubilden, so dass sich das Sperrelement 21 bereits bei der geringsten Aufwärtsbewegung an der Kolbenstange 13 verhakt. Entsprechend rau kann auch die dem Innenraum 27 zugewandte Innenseite des Sperrelements 21 ausgebildet werden, um die Verhakungskräfte, damit die Sicherungskraft, zu erhöhen.

Aus den Erläuterungen zur Funktion der Sicherung 19, insbesondere des Sperrelements 21, wird deutlich, dass die Sicherung 19 auch mehrere Sperrelemente - also beispielsweise Ringe - aufweisen kann, die gemeinsam mit der Kolbenstange 13 in dem hier besprochenen Sinne zusammenwirken und ein Zurückgleiten der Kolbenstange 13 und damit des Verschlussstopfens 7 verhindern.

## Patentansprüche

1. Spritze mit einem Spritzenzylinder (3) und mit einem im Spritzenzylinder (3) beweglichen Verschlussstopfen (7) und mit einer mit diesem zusammenwirkenden Kolbenstange (13), mit einer Sicherung (19), die ein Zurückgleiten des Verschlussstopfens (7) im Spritzenzylinder (3) in jeder Position verhindert, die dieser im Spritzenzylinder (3) einnimmt, und mit einem Sperrelement (21), das an der Kolbenstange (13) in jeder Position angreift, die diese im Spritzenzylinder (3) einnimmt, **dadurch gekennzeichnet, dass** das Sperrelement (21) an einer Halterung (25) so angebracht ist und von dieser so absteht, dass ein Anlenkpunkt (26) des Sperrelements (21) an der Halterung (25) näher am Verschlussstopfen (7) liegt als das dem Anlenkpunkt (26) gegenüberliegende freie Ende (31) des Sperrelements (21).

2. Spritze nach Anspruch 1, **dadurch gekennzeichnet, dass** das Sperrelement (21) an einer Halterung (25) so angebracht ist und von dieser so absteht, dass der Anlenkpunkt (26) des Sperrelements (21) an der Halterung (25) näher am Verschlussstopfen (7) liegt als das dem Anlenkpunkt (26) gegenüberliegende freie Ende (31) des Sperrelements (21).

3. Spritze nach Anspruch 2, **dadurch gekennzeichnet, dass** die Halterung (25) das Sperrelement (21) ausschließlich am Anlenkpunkt (26) hält, sodass das Sperrelement (21) frei um den Anlenkpunkt (26) beweglich ist.

4. Spritze nach Anspruch 2 oder 3, **dadurch gekennzeichnet, dass** die Halterung (25) - in Richtung des Verlagerungsweges des Verschlussstopfens (7) und der Kolbenstange (13) - nachgiebig ausgebildet ist.

5. Spritze nach Anspruch 4, **dadurch gekennzeichnet, dass** die Halterung (25) einen gewellten Steg aufweist.

6. Spritze nach einem der Ansprüche 2 bis 5, **dadurch gekennzeichnet, dass** die Halterung (25) an einem Verschlusselement (16) für den Spritzenzylinder (3) anbringbar ist.

7. Spritze nach Anspruch 5, **dadurch gekennzeichnet, dass** das Verschlusselement (16) als Backstop ausgebildet ist, der ein Herausziehen des Verschlussstopfens (7) aus dem Spritzenzylinder (3) verhindert.

8. Spritze nach Anspruch 6 oder 7, **dadurch gekennzeichnet, dass** das Verschlusselement (16) als Fingerauflage (17) ausgebildet ist.

9. Spritze nach einem der vorhergehenden Ansprüche, **dadurch gekennzeichnet, dass** das Sperrelement (21) verkippbar ist und beim Einschieben der Kolbenstange (13) in den Spritzenzylinder (3) eine Freigabeposition und bei einer entgegengesetzten Bewegung der Kolbenstange (13) eine Verriegelungsposition einnimmt, wobei das Sperrelement (21) mit der Mittelachse (23) der Kolbenstange (13) einen Winkel < 90° einschließt, der in der Freigabeposition größer ist als in der Verriegelungsposition.

10. Spritze nach einem der vorhergehenden Ansprüche, **dadurch gekennzeichnet, dass** das Sperrelement (21) mit einer Vorspannkraft beaufschlagbar ist, die es in die Verriegelungsposition drängt.

11. Spritze nach Anspruch 9, **dadurch gekennzeichnet, dass** das Sperrelement (21) in einer definierten Winkel-Position fest an der Halterung (25) angebracht ist und von dieser in die definierte Position gedrängt wird.

12. Spritze nach einem der vorhergehenden Ansprüche, **dadurch gekennzeichnet, dass** zwischen dem Sperrelement (21) und der Außenfläche der Kolbenstange (13) Reibungskräfte wirken, aufgrund derer das Sperrelement (21) bei einer Verlagerung der Kolbenstange (13) in Richtung auf den Nadelansatz (9) in seine Freigabeposition verlagert wird.

13. Spritze nach einem der vorhergehenden Ansprüche, **dadurch gekennzeichnet, dass** das Sperrelement (21) die Kolbenstange (13) so umgreift, dass es in der Verriegelungsposition an zwei Stellen mit der Kolbenstange (13) zusammenwirkt.

14. Spritze nach einem der vorhergehenden Ansprüche, **dadurch gekennzeichnet, dass** das Sperrelement (21) als Ring ausgebildet ist.

15. Spritze nach einem der vorhergehenden Ansprüche, **dadurch gekennzeichnet, dass** ein Entsperrelement (33) vorgesehen ist, mit dessen Hilfe das Sperrelement (21) in die Freigabeposition verlagerbar ist und/oder in dieser gehalten werden kann.

16. Spritze nach einem der vorhergehenden Ansprüche, **dadurch gekennzeichnet, dass** das Entsperrelement (33) an dem freien Ende (31) des Sperrelements (21) eingreift.

17. Spritze nach einem der vorhergehenden Ansprüche, **dadurch gekennzeichnet, dass** das Entsperrelement (33) aus dem Inneren des Spritzenzylinders (3) über das Verschlusselement (16) hinausragt.

## Claims

1. A syringe with a syringe cylinder (3) and with a sealing plug (7) movable within the syringe cylinder (3), and with a plunger (13) cooperating with the same, with a safety device (19), which prevents a sliding back of the sealing plug (7) within the syringe cylinder (3) in any position the same assumes within the syringe cylinder (3), and with a locking element (21), which engages at the plunger (13) in any position the same assumes within the syringe cylinder (3), **characterized in that** the locking element (21) is attached to a retaining fixture (25), and protrudes from the same in such a manner that an attachment point (26) of the locking element (21) is closer to the sealing plug (7) at the retaining fixture (25) than the free end (31) of the locking element (21) opposite of the attachment point (26).

2. The syringe according to claim 1, **characterized in that** the locking element (21) is attached to a retaining fixture (25), and protrudes from the same in such a manner that the attachment point (26) of the locking element (21) is located closer to the sealing plug (7) at the retaining fixture (25) than the free end (31) of the locking element (21) opposite of the attachment point (26).

3. The syringe according to claim 2, **characterized in that** the retaining fixture (25) retains the locking element (21) exclusively at the attachment point (26) so that the locking element (21) is freely movable around the attachment point (26).

4. The syringe according to claims 2 or 3, **characterized in that** the retaining fixture (25) - in the direction of the displacement path of the sealing plug (7) and of the plunger (13) - is embodied in a flexible manner.

5. The syringe according to claim 4, **characterized in that** the retaining fixture (25) has a wavy bar.

6. The syringe according to one of the claims 2 to 5, **characterized in that** the retaining fixture (25) can be attached to a sealing element (16) for the syringe cylinder (3).

7. The syringe according to claim 5, **characterized in that** the sealing element (16) is embodied as a backstop, which prevents the pulling out of the sealing plug (7) from the syringe cylinder (3).

8. The syringe according to claims 6 or 7, **characterized in that** the sealing element (16) is embodied as a finger impression piece (17).

9. The syringe according to one of the previous claims, **characterized in that** the locking element (21) can be tilted, and assumes a releasing position when the plunger (13) is pushed into the syringe cylinder (3), and a locking position when the plunger (13) is moved in the opposite direction, wherein the locking element (21) encompasses an angle of < 90° with the center axis (23) of the plunger (13), which is larger in the releasing position than in the locking position.

10. The syringe according to one of the previous claims, **characterized in that** the locking element (21) can be impinged with a pretensioning force, which forces the same into the locking position.

11. The syringe according to claim 9, **characterized in that** the locking element (21) is firmly attached to the retaining fixture (25) in a defined angular position, and is forced into the defined position by the same.

12. The syringe according to one of the previous claims, **characterized in that** friction forces are exerted between the locking element (21) and the exterior of the plunger (13), due to which the locking element (21) is displaced into its releasing position with a displacement of the plunger (13) in the direction of the needle attachment piece (9).

13. The syringe according to one of the previous claims, **characterized in that** the locking element (21) encompasses the plunger (13) in such a manner that it cooperates with the plunger (13) in the locking position at two locations.

14. The syringe according to one of the previous claims, **characterized in that** the locking element (21) is embodied as a ring.

15. The syringe according to one of the previous claims, **characterized in that** an unlocking element (33) is provided, with the aid of which the locking element (21) can be displaced into the releasing position and/or can be retained in the same.

16. The syringe according to one of the previous claims, **characterized in that** the unlocking element (33) engages at the free end (31) of the locking element (21).

17. The syringe according to one of the previous claims, **characterized in that** the unlocking element (33) protrudes from the interior of the syringe cylinder (3) over the sealing element (16).

## Revendications

1. Seringue avec un cylindre de seringue (3) et avec un bouchon de fermeture (7) mobile dans le cylindre de seringue (3) et avec une tige de piston (13) coopérant avec celui-ci, avec une sécurité (19) qui empêche un retour de coulissement du bouchon de fermeture (7) dans le cylindre de seringue (3) dans chaque position que prend celui-ci dans le cylindre de seringue (3), et avec un élément de blocage (21) qui s'applique sur la tige de piston (13) dans chaque position que prend celle-ci dans le cylindre de seringue (3), **caractérisée en ce que** l'élément de blocage (21) est monté sur un support (25) et dépasse de celui-ci, de telle sorte qu'un point d'articulation (26) de l'élément de blocage (21) sur le support (25) se situe plus près du bouchon de fermeture (7) que l'extrémité libre (31) de l'élément de blocage (21) opposée au point d'articulation (26).

2. Seringue selon la revendication 1, **caractérisée en ce que** l'élément de blocage (21) est monté sur un support (25) et dépasse de celui-ci, de telle sorte que le point d'articulation (26) de l'élément de blocage (21) sur le support (25) se situe plus près du bouchon de fermeture (7) que l'extrémité libre (31) de l'élément de blocage (21) opposée au point d'articulation (26).

3. Seringue selon la revendication 2, **caractérisée en ce que** le support (25) maintient l'élément de blocage (21) exclusivement au point d'articulation (26), de sorte que l'élément de blocage (21) est librement mobile autour du point d'articulation (26).

4. Seringue selon la revendication 2 ou 3, **caractérisée en ce que** le support (25) est réalisé de manière flexible en direction du trajet de déplacement du bouchon de fermeture (7) et de la tige de piston (13).

5. Seringue selon la revendication 4, **caractérisée en ce que** le support (25) présente une traverse ondulée.

6. Seringue selon l'une quelconque des revendications 2 à 5, **caractérisée en ce que** le support (25) peut être monté sur un élément de fermeture (16) pour le cylindre de seringue (3).

7. Seringue selon la revendication 5, **caractérisée en ce que** l'élément de fermeture (16) est réalisé en tant qu'antirecul qui empêche une extraction du bouchon de fermeture (7) du cylindre de seringue (3).

8. Seringue selon la revendication 6 ou 7, **caractérisée en ce que** l'élément de fermeture (16) est réalisé en tant qu'appui pour doigt (17).

9. Seringue selon l'une quelconque des revendications précédentes, **caractérisée en ce que** l'élément de blocage (21) peut être basculé et prend une position de déblocage lors de l'insertion de la tige de piston (13) dans le cylindre de seringue (3) et une position de verrouillage lors d'un mouvement opposé de la tige de piston (13), dans laquelle l'élément de blocage (21) inclut un angle < 90° avec l'axe central (23) de la tige de piston (13) qui est plus grand dans la position de déblocage que dans la position de verrouillage.

10. Seringue selon l'une quelconque des revendications précédentes, **caractérisée en ce que** l'élément de blocage (21) peut être sollicité avec une force de précontrainte qui le pousse dans la position de verrouillage.

11. Seringue selon la revendication 9, **caractérisée en ce que** l'élément de blocage (21) est monté de manière fixe sur le support (25) dans une position angulaire définie et est poussé de celle-ci dans la position définie.

12. Seringue selon l'une quelconque des revendications précédentes, **caractérisée en ce que** des forces de frottement agissent entre l'élément de blocage (21) et la surface extérieure de la tige de piston (13), forces en raison desquelles l'élément de blocage (21) est déplacé dans sa position de déblocage lors d'un déplacement de la tige de piston (13) en direction de l'embouchure d'aiguille (9).

13. Seringue selon l'une quelconque des revendications précédentes, **caractérisée en ce que** l'élément de blocage (21) entoure la tige de piston (13), de telle sorte qu'il coopère avec la tige de piston (13) en deux endroits dans la position de verrouillage.

14. Seringue selon l'une quelconque des revendications précédentes, **caractérisée en ce que** l'élément de blocage (21) est réalisé en tant qu'anneau.

15. Seringue selon l'une quelconque des revendications précédentes, **caractérisée en ce qu'**un élément de déblocage (33), à l'aide duquel l'élément de blocage (21) peut être déplacé dans la position de déblocage et/ou peut être maintenu dans celle-ci, est prévu.

16. Seringue selon l'une quelconque des revendications précédentes, **caractérisée en ce que** l'élément de déblocage (33) s'engrène sur l'extrémité libre (31) de l'élément de blocage (21).

17. Seringue selon l'une quelconque des revendications précédentes, **caractérisée en ce que** l'élément de déblocage (33) dépasse de l'intérieur du cylindre de seringue (3) au-dessus de l'élément de fermeture (16).
